# EUROPEAN PATENT APPLICATION

(11) **EP 0 627 173 A1**
(43) Date of publication of application: **07.12.1994**
(21) Application number: 94201476.2
(22) Date of filing: 26.05.1994
(51) Int. Cl.: A23L 1/29, A23P 1/04, A61K 9/50

(54) **Preparation for dietetic use or as a food supplement, with targeted release in the colon**

(30) Priority: 31.05.1993 IT MI931131
(71) Applicant: GIULIANI S.p.A., I-20129 Milano (IT)
(72) Inventor: Giorgetti, Enzo, Milano (IT); Brunetti, Gabriele, 20129 Milano (IT); Marchioretto, Danila, 20099 Sesto San Giovanni(Milano) (IT); Frigerio, Giuliano, 20020 Arese (Milano) (IT)
(74) Representative: Appoloni, Romano

(57) **Abstract**

The invention proposes a preparation for dietetic use or as a food supplement, characterised by comprising a nucleus or an assembly of nuclei, each comprising one or more nutritional substances such as vitamins, mineral salts, oligoelements, enzymes, amino acids, phospholipids, fatty acids or constituent elements of physiological bacterial flora, the nucleus being coated with at least one covering layer of a macromolecular substance consisting of cellulose or a derivative or analogue thereof, for the substantially total release of said nutritional substances into the colon.

## Description

The colon is known to be the seat of the final stage of metabolic-catabolic processes, and being an organ very rich in microorganisms and responsible for forming the fecal mass must be able to operate constantly at optimum level because otherwise it could frequently become the seat of heterogeneous and sometimes very serious proteiform pathological symptoms, such as functional alterations, constipation or diarrhea symptoms, polyposis, diverticulosis, local or diffuse ectasia, dysplasia, carcinoma, IBD etc.

The relationship between colon functionality/intestinal transit/physiological fecal mass formation and the problematic biochemistry and metabolism which govern the terminal part of the digestive tract is very close, to the extent that often the type of food determines the appearance or persistence of alterations of various kinds.

The colon is not only the terminal organ of transit in the digestive tract, but is also the main metabolic organ of the body, because of the presence of intestinal bacterial flora very rich in different kinds of biological activity.

Because it is also a metabolic organ, the colon represents the seat of production of an important quota of free radicals and a obligatory crossing path for entry into the body of catabolic toxic products or the refuse from the metabolism of different nutrients.

It is known that specific acaloric micronutrients (vitamins, oligoelements, enzymes, nucleotides etc.) or certain elementary compounds (such as essential amino acids) exercise key roles (of essential type) in determined stages of metabolic processes in general, and it is also known that many metabolic processes take place in the colon.

The metabolic processes which take place in the colon (lumen and colocolic mucosa) are of various types, but they can be divided essentially into two large groups, namely: metabolic processes regarding hydroelectrolytic lumen/tissue liquid/blood interchange, and the absorption of terminal fractions of the various primary metabolisms (glucides, protides, lipids) or the reabsorption of substances concerning entero-hepatic circulation (such as metabolized bile salts), with significance as detoxication processes; and the production of toxic factors and systems for their elimination, in which context the production of ammonium radicals (highly damaging to cerebral structures) as the terminal process in nitrogenated metabolism and the production of potentially carcinogenic substances etc. are particularly important in relation to their potential toxicity. It should be noted that precisely in relation to the mentioned factors, cancer of the colon is among the most frequent tumors, it being second only to lung cancer. In addition, the frequency of cancer of the colon becomes progressively higher in passing from the proximal segments to the more distal segments (the most frequent are tumors of the sigmoid and rectum), indicating that agents which form in the lumen of the colon have an extremely important favouring significance.

It is also known that intestinal bacterial flora performs an extremely important role in the numerous biochemical processes which take place in the colon, given that the many bio-enzymatic activities of this flora result in important metabolic modifications in the substrates with which they come into contact. It can be useful to note in this respect that bacterial flora is able to degrade certain food substrates which cannot be attacked or digested by the body digestive enzymes.

Responsibility for many of the events toxic to the body and for the basic aging phenomena is currently attributed to the formation of free radicals, this formation being of a general character in the various body tissues. However it seems certain that the formation of free radicals within the colon structure is particularly high, aided by the biological intervention of intestinal bacterial flora.

Equilibrium between the production of free radicals and antioxidant biological mechanisms represents an essential condition for maintaining a state of well being. An increase in free radical production should result in a corresponding increase in detoxication processes, whereas with increasing age there is a reduction in the efficiency of these processes.

The idea of concentrating within the colon micronutrients able to more efficiently (and physiologically) control the metabolic stages and processes taking place in the colon represents the basis for the main object of the present invention.

Oral ingestion of such micronutrients normally results in considerable absorption in the first part of the digestive tract (stomach, duodenum, ileum), with the result that their direct presence in the colon is practically inexistent or negligible.

The object of the present invention is to prevent or hinder a too premature systemic absorption, to hence enable the dietetic-nutritional principles in question (essential micronutrients) to reach the colon in high concentration.

Suitable methods for controlling the release of active principles after oral administration ore widely known in drug production, starting with the so-called gastro-protected forms.

Specifically, pharmaceutical gastro-protected or colon-targeted release forms can be achieved using various methods based on a plurality of technical adjuvants.

This release can be dependent on time or on the pH of the intestinal lumen, and the procedures generally used involve coating the pharmaceutical forms with polymers of different types.

There are however no described specific methods of alimentary type applied to dietetic nutrients for substantially the same purpose, ie to prevent premature absorption of micronutrients so that they reach physiological regions where their presence in a higher dosage would appear more useful.

The typical adjuvants for controlled release of drugs after oral administration are in most cases not permitted for dietetic food products, without considering the fact that food supplement doses are normally much higher than drugs.

This object and further advantages which will be apparent hereinafter are attained according to the present invention by a preparation for dietetic nutritional use or for use as a food supplement in accordance with claim 1.

Said nucleus can be a single nucleus, or consist of an assembly of separate micronuclei, each with its own coating.

Nutritional substances which can form part of the nucleus of the preparation according to the invention are generally vitamins, mineral salts, oligoelements, enzymes, amino acids, phospholipids (soya lecithin), fatty acids, constituent elements of physiological bacterial flora (lactic ferments, etc.), nucleotides, etc. These are substances all to a certain degree essential to the functionality of the digestive tract and/or in the various metabolic paths and in the formation/regeneration of tissues, but of which the primary purpose is not to provide caloric energy.

They comprise all the vitamins, with particular reference to vitamins of specific antioxidant action (vitamins A, C, E) and vitamins of group B, the various minerals and oligoelements the ingestion levels of which are recommended on the basis of specific studies (again in particular those related to mechanisms involving detoxication from the accumulation of free radicals, such as copper, zinc, manganese, selenium, magnesium etc.), the various nucleic fractions and enzymatic principles of natural origin present as such or in mixed form in extractive foods, soya lecithin, aspartame, amino acids, etc.

They can comprise either compounds/products of orthomolecular type (ie already forming part of the human body, as is the case with most of the aforelisted), or non-orthomolecular products of natural vegetable or animal nature, but always with the character of a nutraceutic food, ie with a specific scientific connotation in relation to particular metabolic stages.

The layer covering the nucleus of the composition of the invention comprises at least one macromolecular substance consisting of cellulose or a derivative or analogue thereof. These are macromolecules indigestible or poorly digestible by the gastro-enteric juices but potentially degradable by the enzymatic activity of intestinal bacterial flora, and legally permitted on foods, such as:
- microcrystalline cellulose
- methylcellulose
- hydroxypropylcellulose
- hydroxypropylmethylcellulose
- methylethylcellulose
- carboxymethylcellulose

This covering layer can also comprise other substances, such as those suitable for forming covering layers for food use, such as xanthan gum, alginic acid and alginates, agar agar, carrageenin, carob seed flour, guar seed flour, tragacanth, gum arabic, pectin, lac, hydrogenated vegetable fat, dextrose, saccharose etc.

According to the invention, a series of clinical observations using the breath test and/or hematochemical analysis gave results of surprising interest.

For example, it was observed that in subjects administered with a preparation consisting of antioxidant and acidifying nutraceutics coated with macromolecules based on mass-forming indigestible fibres useful for allowing or improving the formation of a physiological fecal mass and for activating intestinal motility, surprising effects were noted on certain general disturbances and in particular on intestinal pathology symptoms of various origin and with different symptomatological manifestations.

In particular, it was observed that if mixtures of antioxidant vitamins (vitamins A, E and C) with certain oligoelements strictly related to antioxidant processes or more generally to detoxication processes (copper, zinc, manganese, selenium etc.) are ingested in cellulose macromolecular coverings, clinical effects appear within a relatively short time which normally are not found in a normal examination; in particular, colic pain phenomenology, dependent on spastic inflammatory or functional events in the large intestine, appeared to respond with surprising evidence to these nutritional combinations.

Abnormal intestinal bad-odour gas accumulations, a typical consequence of an excess of putrefactive phenomena in the colon, underwent a substantial reduction much appreciated by the subject, when the nucleus of the inventive composition consisted of acidifying nutritional agents of lactobacillar type (Lactobacillus acidophilus, B. subtilis or other agents of the streptococcus lactis group, etc.). Their effectiveness in controlling the said paraphysiological manifestations was very apparent.

Observations made by determining ammonemia levels and by using a toxemia test (Limulus test) showed an appreciable response also from the biochemical viewpoint when vitamin nutraceutics and acidifying bionutritional principles, contained in absorbent fibre coverings, were administered to the subjects. These trials had their main response in subjects with intestinal alterations or with chronic hepatopathies with a cirrhotic imprint, where there is already a tendency to accumulate toxins of intestinal origin deriving from putrefactive degradative metabolism of protein substances with the formation of ammonium compounds.

The breath test carried out on particular subjects with prevalent fermentation or intestinal putrefacfion processes gave indications of fairly evident clinical improvement modifications.

The observations made also appeared interesting in relation to the fact that the indigestible macromolecules based on fibres of various types or more generally the mass-forming preparations, provided their normal clinical effect in terms of aiding the formation of a fecal mass of physiological characteristics in the colon.

In particular, it can be observed that saccharomyces (brewers yeast) and many physiological lactobacilli are inactivated by prolonged presence in an acid pH environment, to disperse along long tracts prior to the colon, where instead their biological action is of greatest necessity.

Iron and other minerals can cause gastric intolerance if not ingested together with food or other gastro-protective elements.

Some non-limiting examples of the present invention are reported hereinafter.

### EXAMPLE 1

| Nucleus composition for a covered tablet: | |
|---|---|
| - ascorbic acid (vitamin C) | 60 mg |
| - α-tocopherol (vitamin E) | 15 mg |
| - β-carotene (pro-vitamin A) | 6 mg |
| - zinc (as oxide) | 18 mg |
| - copper (as gluconate) | 3 mg |
| - chromium (as chloride) | 0.1 mg |
| - iodine | 0.15 mg |
| - mannitol | 10-100 mg |
| - magnesium stearate | 1-2 mg |
| Covered with: | |
| - hydroxypropylcellulose | 10-100 mg |
| - xanthan gum | 5-50 mg |
| - microcrystalline cellulose | 10-100 mg |

### EXAMPLE 2

| Nucleus composition for a covered tablet: | |
|---|---|
| - vitamin B1 | 1.5 mg |
| - vitamin B2 | 2 mg |
| - vitamin B6 | 1.8 mg |
| - vitamin C | 50 mg |
| - pantothenic acid | 6 mg |
| - vitamin E | 15 mg |
| - lactose | 10-100 mg |
| - magnesium stearate | 1-2 mg |
| Covered with: | |
| - saccharose | 10-50 mg |
| - gum arabic | 1-10 mg |
| - sodium carboxymethylcellulose | 10-100 mg |

### EXAMPLE 3

| Nucleus composition for a tablet: | |
|---|---|
| - vitamin A | 0.1 mg |
| - vitamin C | 60 mg |
| - vitamin E | 10 mg |
| - selenium | 0.1 mg |
| - manganese | 3.5 mg |
| - zinc | 18 mg |
| - copper | 3 mg |
| - dextrose | 10-100 mg |
| - magnesium stearate | 1-2 mg |
| Covered with: | |
| - hydrogenated vegetable fats | 10-100 mg |
| - microcrystalline cellulose | 10-100 mg |

### EXAMPLE 4

| Granulate composition in a sachet: | |
|---|---|
| - brewers yeast or Saccharomyces cerevisiae | |
| - Lactobacillus acidophilus | |
| - B.bifidus | |
| - Lactobacillus bulgaricus | |
| - Streptococcus lactis in a total of 1-10 bio of cfu | |
| - vitamin B1 | 1.2 mg |
| - vitamin B2 | 1.8 mg |
| - vitamin B6 | 1.5 mg |
| - vitamin C | 50 mg |
| - pantothenic acid | 6 mg |
| - vitamin E | 10 mg |
| Covered with: | |
| - methylcellulose to make up to | 2-5 g |

### EXAMPLE 5

| Granulate composition in a sachet: | |
|---|---|
| - taurine | 1 g |
| - soya lecithin | 1 g |
| - vitamin A | 0.1 mg |
| - vitamin C | 60 mg |
| - vitamin E | 5 mg |
| - zinc | 18 mg |
| - copper | 3 mg |
| - manganese | 2 mg |
| - aspartame | 10 mg |
| Covered with: | |
| - pectin | 10-100 mg |
| - hydroxypropylmethylcellulose to make up to | 3-5 g |

### EXAMPLE 6

| Microgranulate composition in a sachet: | |
|---|---|
| - taurine | 1 g |
| - bilberry anthocyanic complex | 1 g |
| - vitamin A | 0.1 mg |
| - vitamin B1 | 1.2 mg |
| - vitamin B2 | 1.5 mg |
| - vitamin B6 | 1.8 mg |
| - vitamin C | 60 mg |
| - vitamin E | 5 mg |
| - zinc | 18 mg |
| - copper | 3 mg |
| - iodine | 0.15 mg |
| - manganese | 2 mg |
| - aspartame | 10 mg |
| Covered with: | |
| - guar seed flour | 10-100 mg |
| - methylethylcellulose to make up to | 3-5 g |

### EXAMPLE 7

| Microgranulate composition in a sachet: | |
|---|---|
| - taurine | 1 g |
| - vitamin A | 1 mg |
| - vitamin B6 | 1.8 mg |
| - vitamin C | 60 mg |
| - vitamin E | 10 mg |
| - zinc | 18 mg |
| - copper | 3 mg |
| - manganese | 3 mg |
| Covered with: | |
| - microcrystalline cellulose | 10-100 mg |
| - lac | 10-100 mg |

### EXAMPLE 8

| Composition for 1 or 2 covered tablets or microgranulate in a sachet: | |
|---|---|
| - taurine | 1 g |
| - vitamin B6 | 1.8 mg |
| - vitamin E | 10 mg |
| - mannitol | 50-200 mg |
| - magnesium stearate | 5-20 mg |
| Covered with: | |
| - microcrystalline cellulose | 10-100 mg |
| - hydrogenated vegetable fat | 50-500 mg |

### EXAMPLE 9

| Composition for a capsule of covered microgranules: | |
|---|---|
| - butyric acid | 200 mg |
| - vitamin A | 0.1 mg |
| - vitamin C | 60 mg |
| - vitamin E | 10 mg |
| - selenium | 0.1 mg |
| - manganese | 3.5 mg |
| - zinc | 18 mg |
| - copper | 3 mg |
| - gelatin | 60-120 mg |
| - lactose | 50-200 mg |
| - magnesium stearate | 2-5 mg |
| Covered with: | |
| - methyl cellulose | 10-50 mg |
| - methylethylcellulose | 10-50 mg |

In general, the compositions of the present invention can be prepared typically in food form, such as wafers, granulates for making up suspensions to be drunk etc., or parapharmaceutic form such as covered tablets, capsules containing covered microgranules, sachets, pills etc., which are often better for regular continuous use of the product.

## Claims

1. A preparation for dietetic use or as a food supplement, characterised by comprising a nucleus or an assembly of nuclei, each comprising one or more nutritional substances such as vitamins, mineral salts, oligoelements, enzymes, amino acids, phospholipids, fatty acids or constituent elements of physiological bacterial flora, the nucleus being coated with at least one covering layer of a macromolecular substance consisting of cellulose or a derivative or analogue thereof, for the substantially total release of said nutritional substances into the colon.

2. A preparation as claimed in claim 1, characterised in that said nutritional substances included in said nucleus are chosen from the following: vitamins A, C, E B-group vitamins, pantothenic acid, vitamin complexes; mineral salts and oligoelements such as zinc, copper, iodine, manganese, selenium, chromium; brewers yeast; lactic ferments; amino acids; aspartame; butyric acid.

3. A preparation as claimed in claim 1, characterised in that said macromolecular substance in the covering layer is chosen from microcrystalline cellulose; methylcellulose; hydroxypropyl cellulose; hydroxypropylmethylcellulose; methylethylcellulose; carboxymethylcellulose, either as such or in mixture with each other.

4. A preparation as claimed in claim 1, characterised in that said covering layer comprises a further component chosen from xanthan gum, alginic acid and alginates, agar agar, carrageenin, carob seed flour, guar seed flour, tragacanth, gum arabic, pectin, lac, hydrogenated vegetable fat, dextrose, saccharose, either as such or in mixture with each other.
